# EUROPEAN PATENT SPECIFICATION

(11) **EP 4 075 981 B1**
(45) Date of publication and mention of the grant of the patent: **04.11.2026**
(21) Application number: 20824550.6
(22) Date of filing: 15.12.2020
(51) Int. Cl.: A01N 43/80, A61K 31/42, A01P 7/04, A61K 31/4709, A61K 31/4725

(54) **COMPOSITION FOR LICE CONTROL**
ZUSAMMENSETZUNG ZUR LÄUSEBEKÄMPFUNG
COMPOSITION DE LUTTE CONTRE LES POUX

(30) Priority: 16.12.2019 EP 19216377
(43) Date of publication of application: 26.10.2022
(73) Proprietor: Intervet International B.V., 5831 AN Boxmeer (NL)
(72) Inventor: O'NEILL, Peter, Andrew, Macquarie Park, NSW 2113 (AU); FISARA, Petr, Macquarie Park, NSW 2113 (AU); FLOCHLAY-SIGOGNAULT, Annie, Madison, New Jersey 07940 (US); HUYGHE, Bruno, 49071 Beaucouzé Cedex (FR)
(74) Representative: Intervet International B.V.
(86) International application number: PCT/EP2020/086097
(87) International publication number: WO 2021/122513

(56) References cited:
- EP-A1- 2 875 726
- WO-A1-2013/150052
- WO-A1-2015/086551
- WO-A1-2016/138339
- WO-A1-2019/115492
- WO-A1-2019/122324
- WO-A2-2009/024541

## Description

### Field of the invention

The present invention relates to the treatment or prevention of parasitic arthropod infestations of animals.

### BACKGROUND OF THE INVENTION

Sheep lice are a major problem mainly to the Australian and New Zealand sheep industry.

Such lice infesting sheep are typically categorized into those that feed directly on blood of the host sheep - sucking lice and those that feed on dead skin, secretions and bacteria normally found at the surface of the skin - biting or chewing lice. Three species of sucking lice are known on sheep *- Linognathus pedalis, L. africanus* and *L. ovillus.*

However, the biting or chewing louse- the Sheep body louse, *Bovicola ovis,* is the most prevalent and economically important louse species infesting sheep in all major sheep producing countries including Australia and New Zealand. Sheep body lice are obligate parasites and complete their whole life cycle on the sheep. They feed on dead skin, secretions and bacteria normally found at the surface of the skin and move within the fleece of sheep depending on the temperature.

The main source of infestation of sheep is from contact with infested sheep. Infestations are spread via direct contact, such as when sheep are penned in sheep yards or woolsheds or congregate in rest/shade areas.

The size of the lice population on individual sheep fluctuates during the year according to the season and shearing time. The lice population is smallest immediately after summer shearing and peaks in mid-winter. Shearing significantly reduces the size of the lice population by physical removal and subsequent exposure to environmental conditions. Strong ultraviolet light and very high fleece temperatures reduce lice populations.

Lice cause irritation in infested sheep inducing rubbing, which results in damaged wool, and poor wool growth, as well as discomfort and annoyance to the animal. In addition, blowflies may strike damaged and moist areas of wool of infested animals.

Lice Infestations can cause significant production losses in sheep in two ways:
1. Fleece damage: Lice cause intense irritation. Infested sheep bite themselves or rub against trees and fences, leading to fleece derangement, cotting or broken fibres. Lousy wool often has a yellow color and a distinctive smell caused by skin secretions, causing further downgrading.
2. Hide damage: Lice can trigger an immune response known as cockle, which causes thickening of the skin and pelt damage.

Apart from the direct economic costs, the continued use of conventional treatments to control lice infestation can have detrimental effects through residues from such treatment (especially residual dip or other fluids containing pesticide residues) entering the environment and food chain as well as on farmer safety.

Conventional methods of lice control include chemicals that are applied as dips, jetting fluids, sprays and pour-ons, which kill lice by contact. All sheep body lice treatments currently registered (2019) in the major markets Australia and New Zealand are topically applied treatments with the active ingredients from the following chemical classes: Neonicotinoid (Imidacloprid, Thiacloprid), Spinosyn (Spinosad), Organophosphate (OP: temephos, diazinon), Macrocyclic lactone (ML: abamectin, ivermectin), Magnesium fluorosilicate/sulphur (MgFSi, sulphur, rotenone), Insect growth regulator (IGR: diflubenzuron, triflumuron), and Synthetic pyrethroid (SP: cypermethin, alphacypermethrin, deltamethrin).

Such products are applied by the following conventional topical methods.

Backline applications: Relatively small volumes of product are applied along the backs of the sheep from head to rump. From here, the chemical spreads over the surface of the body, assisted by contact between sheep.

Immersion (plunge and cage) dipping: In this method of treatment, sheep are completely immersed in dipping solution. Effective lice control relies on sheep being wet to skin level over their entire body; this can be very difficult because of the waterproofing effect of wool.

Shower dipping: Shower dipping relies on applying a high volume of dip wash from spray nozzles on a slowly rotating boom to completely wet the sheep.

Hand-jetting: Hand-jetting is one method used for applying a lice treatment in long wool (greater than 6 weeks wool growth). Hand-jetting requires high pressures at the hand-piece for the liquid to penetrate the wool.

However, lice problems often reoccur in affected sheep flocks after treatment because small populations of surviving lice start to reproduce and farmers may notice sheep scratching, biting, pulled wool, or an infestation at shearing time. Hence, because of the limited efficacy of the conventional topical administration methods frequent re-treatment is required that requires additional handling of the animals, increases the use of pesticides and harms the environment. Therefore, an easy to apply lice treatment that effectively controls or eliminates existing lice infestation and protects from re-infestation would be very desirable in the sheep industry. International patent applications WO 2013/184006 and WO 2014/051440 of Bayer suggest to internally/ systemically administer spinosyn compounds (Spinosad) or neonicotinoid compounds (thiacloprid) for the control of chewing lice. However, no long-term effective treatment of chewing lice infestation has been shown in the documents even under laboratory conditions and multiple high doses need to be administered over time to show a sufficient treatment effect and no protective effect against new lice infestation was demonstrated. Up to now no product using oral administration to sheep for the control of biting/ chewing lice has been introduced in any market. Therefore, none of the prior art methods satisfies the needs of the sheep producer. Thus, there is a need in this art to find a better solution to the problem of controlling chewing lice infestations in the sheep industry, while not harming animals, the environment or humans. Isoxazoline compounds and their use to protect animals from certain parasites have been described in the prior art. WO2019/122324 discloses liquid veterinary pour-on compositions for the control of parasite infestations in livestock animals. WO2007/024451 discloses the use of isoxazolines to control parasites of animals. WO2019/115492 discloses implants for the control of parasite infestations in livestock animals. WO2016/138339 discloses long-acting injectable veterinary compositions and their use for the treatment or prevention of a parasite infestation or infection on or in an animal. WO2015/086551 discloses methods for preventing reinfestation of pets by adult fleas. WO2013/150052 discloses soft chewable veterinary products for the administration to pets.

### SUMMARY OF THE INVENTION

Any references to methods of treatment or therapeutic use in the subsequent paragraphs of this description are to be interpreted as references to the compound or composition containing the compound of the present invention for use in a method for treatment of the animal body by therapy.

The invention provides a new method and composition to control chewing/ biting lice of sheep independent of its wool length. Accordingly, in one embodiment the present invention relates to an isoxazoline compound of Formula (I) wherein the isoxazoline compound of formula (I) is fluralaner or a salt or solvate thereof for use in the treatment or protection of sheep from *Bovicola ovis* infestation after a single oral administration of a composition comprising an effective amount of at least one isoxazoline compound of Formula (I) to sheep of any wool length.

In one embodiment the invention as defined in claim 1 provides a composition comprising an effective amount of such compound of Formula (I) or a salt or solvate thereof, when used for the elimination of chewing lice parasite infestations of sheep and/ or for the protection of sheep from infestation by chewing lice, wherein the composition is administered once orally to sheep of any wool length. In another preferred embodiment the composition is administered once more than 6 weeks after shearing.

Another embodiment is a method of treating sheep that are diagnosed or suspected to be infested by chewing lice parasites, comprising administering once orally an effective amount of at least one isoxazoline compound of Formula (I) or a salt or solvate thereof to sheep independent of its wool length.

Another embodiment is a method of protecting sheep from chewing lice infestation comprising once orally administering an effective amount of at least one isoxazoline compound of Formula (I) or a salt or solvate thereof to sheep independent of their wool length.

### BRIEF DESCRIPTION OF THE FIGURES

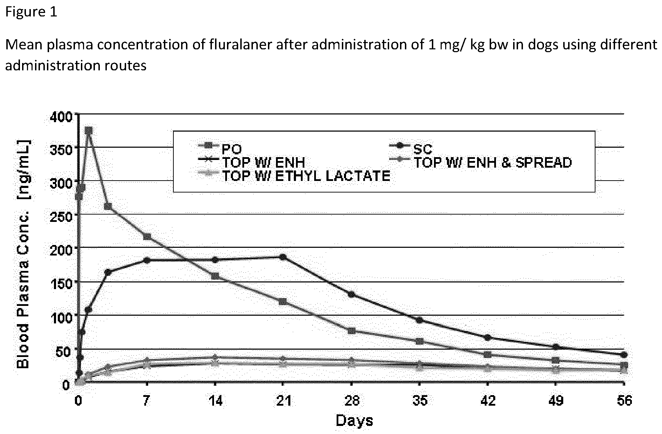

Figure 1 shows as comparison the plasma concentration of fluralaner (compound 11-1) after administration of 1 mg/kg bw to beagle dogs using various administration routes: Group A - PO (oral), Group B - SC (subcutaneous), Group C, D, E - Top - (topical). This figure is taken from WO 2009/024541.

The compositions tested were:

| **Group** | **Treatment** |
|---|---|
| A | *tablet for oral administration.* The composition was 13.33% Compound11-1; 3.29% lactose monohydrate (Granulac 200); 0.01% of sodium lauryl sulfate; 0.90% Povidone 25; 0.15% water; 40.98% lactose monohydrate (Flowlac 100); 33.33% corn starch; 3.00% silica, colloidal anhydrous; 4.00% sodium starch glycolate; and 1.00% magnesium stearate. |
| B | *injectable solution for subcutaneous administration.* One ml of the composition contained 20 mg of Compound 11-1; 0.4 ml of dimethyl sulfoxide;0.24 ml of propylene glycol; and sufficient ethyl lactate to bring the total volume to 1 ml. |
| C | *topical solution for spot-on administration (the solution included an absorption enhancer).* One ml of the composition contained 20 mg of Compound 11-1; 0.35 ml of dimethyl sulfoxide; and sufficient dipropylene glycol monomethyl ether to bring the total volume to 1 ml. |
| D | *topical solution for spot-on administration (the solution included an absorption enhancer and spreading agent).* One ml of the composition contained 20 mg of Compound 11-1; 0.35 ml of dimethyl sulfoxide; 0.1 ml of isopropyl myritate; and sufficient dipropylene glycol monomethyl ether to bring the total volume to 1 ml. |
| E | *topical solution for spot-on administration.* One ml of the composition contained 20 mg of Compound 11-1; and sufficient ethyl lactate to bring the total volume to 1 ml. |

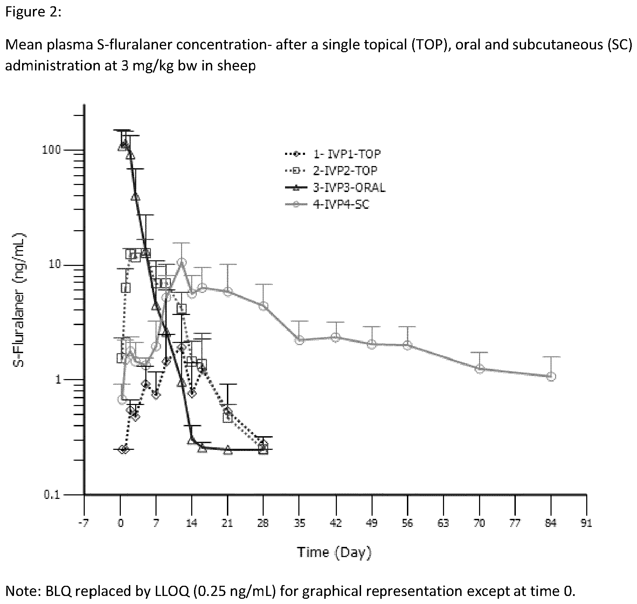

Figure 2 shows mean plasma S-fluralaner concentration- after a single topical, oral and subcutaneous administration at 3 mg/kg bw in sheep.

### DETAILED DESCRIPTION OF THE INVENTION

It is known that lice infestations can only be successfully and sustainably treated in off-shears and short wool sheep, but not in long wool sheep. Existing backline products with long wool treatment claims are available as "emergency treatment" only because they are known to only reduce the lice burden and therefore require a re-treatment of the animal at the next shearing with a different product. Other prior art methods for topical treatment are impractical in long wool sheep (such as immersion and shower dipping) because the long wool prevents effective wetting of the sheep. Therefore, there is a high desire for an effective product that can be used in sheep of all wool lengths, including in long wool.

Isoxazoline compounds of Formula (I) are known to be effective to control blood sucking parasites such as fleas and ticks and that long-term efficacy and blood levels can be obtained in treated animals when using oral administration of such compounds. Figure 1, that was taken from WO 2009/024541, shows blood concentration of fluralaner after administration of 1 mg/kg bw to dogs using different administration routes. Figure 1 shows that in dogs after oral administration effective blood levels for at least 56 days are obtained.

However, it has been found that the pharmacokinetic profile varies between animal species. Background Example and Figure 2 shows plasma concentration of fluralaner after administration of 3 mg/kg bw to sheep using different administration routes. From comparison of Figures 1 and 2, it is clear that the profile is very different when comparable dosages of fluralaner are administered to different species. With respect to oral administration, these graphs show that fluralaner levels in sheep blood abruptly declined below 1 ng/mL by day 12 post-dosing, whereas in dogs (with a lower dose of 1 mg/kg bw), there were still effective levels. By 21 days post-dosing, the concentration of all sheep was below the LLOQ after oral administration.

Based on this pharmacokinetic profile it was very surprising that with a single oral administration of an effective amount of an isoxazoline compound as described in this application effective treatment and protection against non- blood sucking lice in sheep can be obtained.

As it has been shown in the examples, a single administration of a composition comprising an effective amount of isoxazoline compounds of formula (I) as described below, especially fluralaner, by oral administration to sheep can effectively treat existing lice infestations, in long wool sheep and off-shears sheep. Currently, sheep are mostly treated off-shears or in short wool for practical management reasons and due to the difficulties of achieving sufficient efficacy against chewing lice in long wool. Consequently, the use according to the invention provides animal welfare benefits by reducing the time that infested animals will remain untreated and suffer from the discomfort and annoyance caused by the lice infestation.

Additionally, one treatment provides protection of sheep against chewing lice re-infestation as shown in Example 4 in which treated sheep are challenged with lice within 2 weeks after administration and no lice infestation is established on the treated sheep for more than 12 weeks.

In view of the pharmacokinetic profile after oral administration, this was very unexpected because the blood level of the isoxazoline compound fluralaner declined very rapidly after oral administration to sheep so that no sustained effect, that would be necessary for an effective control/ eradication was expected after administration of an oral formulation without the need to employ extended/ slow-release formulation for a sustainable effect in sheep.

The benefits of such a method are that:
a) This method is more convenient than prior art application on animals because it avoids labor intensive techniques and avoids stress in the sheep animals when using such prior art topical methods;
b) furthermore, the prior art application of parasiticides raises concerns in connection to animal safety and user safety during their administration, sheep can be handled immediately after treatment without exposure to the parasiticide;
c) such a method is easy to apply using e.g. available drenching equipment on the sheep farms and therefore requires no or only minimal investments in new equipment; and
d) by this method an administration to a sheep independent of wool length during a defined time period is possible and therefore an effective control of the chewing lice population in a whole sheep flock and production unit even if sequential shearing is used.
e) the resistance breaking properties of such isoxazoline compounds are very favorable i.e. the chewing lice are very susceptible to the inhibiting or killing effect of such isoxazoline compounds
f) generally, only one administration is required to effectively control / eliminate existing chewing lice infestation and no re-treatment of long wool sheep at next shearing would be required and consequently the exposure of the environment to harmful pesticides is reduced.

Consequently, by using the isoxazoline compounds as described in this application disadvantages of the prior art can be avoided, because a single convenient, safer administration of the isoxazoline compound would be sufficient to achieve the desired effect.

Hence the current invention would be a breakthrough in the control of sheep body lice, allowing lice effective control/ elimination of lice from sheep flocks independent of the wool length of the animals.

### Definitions

The following definitions are relevant in connection with the embodiments of the present invention.

All reference in this document to "Lice" or "louse" is meant the parasitic sheep body louse (chewing or biting louse), *Bovicola ovis,* unless specifically indicated otherwise.

By "treating" or "treat" or "treatment" is intended the application or administration of an isoxazoline compound or composition to a sheep animal that has been diagnosed to have a parasitic chewing lice infestation for the eradication of the parasite infestation or the reduction of the number of parasites, infesting the animal. It is a medical care given to animals infested with ectoparasites (WAAVP guidelines 2006)
The effect can be e.g. ovicidal, larvicidal and/or adulticidal or a combination thereof. The effect can manifest itself directly, i.e. killing the parasites either immediately or after some time has elapsed, for example when molting occurs, or by destroying their eggs, or indirectly, e.g. reducing the number of eggs laid and/or the hatching rate.

"Protection" means that a new infestation of the sheep, a sheep mob or flock with chewing lice parasites is prevented by killing adult parasites and any development/larval stages, that are able to infest the host, before infestation of the host or, by killing or inhibiting the parasites when they infest an animal that has been treated with an isoxazoline compound as described before or preventing generation of offspring of the parasites e.g. reducing the number of eggs laid and/or the hatching rate.

Protective periods may be e.g. useful when treated ewes contact untreated lambs, or when treated lambs contact untreated ewes infested with lice or when treated and untreated sheep become co-mingled.

"Protection period" means the time, expressed in days or weeks after the treatment, that a veterinary test product will prevent the ectoparasite re-infestation of the animal hosts. Sometimes referred to as the prophylactic period or the persistent efficacy period (WAAVP guidelines 2006)

As used herein, an "active pharmaceutical ingredient "(or active ingredient, or pharmaceutically active ingredient or pharmaceutically acceptable active ingredient) is a substance used in a pharmaceutical product, intended to furnish pharmacological activity or to otherwise have direct effect in the diagnosis, cure, mitigation, treatment or prevention of disease, or to have direct effect in restoring, correcting or modifying physiological functions in humans or animals.

"Single administration" means that only one dosage of the isoxazoline compound is administered to a sheep.

An "effective amount," is the amount or quantity of an isoxazoline compound as described above that is required to treat or prevent parasitic chewing lice infestations of animals, i.e. to alleviate or reduce lice numbers on an animal, and/or to inhibit the development of parasite infestations on an animal.

This amount is readily determined by observation or detection of the parasite numbers or eggs on the animal both before and after administering an isoxazoline compound as described above to such animals, e.g. the parasite count is reduced, after a first administration, by 5% to about 100%, preferably more than 50%, more than 70%, more than 90%, more than 95%, more than 99.5 % or 100%. Preferably the lice count is reduced by 100%, i.e. the effective amount results in elimination of all lice on a treated animal, i.e. that no chewing lice are found when the sheep are investigated as described above.

Depending on the specific isoxazoline compound used, the administration allows to completely inhibit or kill the chewing lice and preferably all viable eggs present on the animal.

In one embodiment the oral administration of the isoxazoline compound as described above results in an elimination/ eradication of chewing lice infestation. According to regulatory (Australian Pesticides and Veterinary Medicines Authority (APVMA)) guidelines in Australia 'Eradication' is defined as: 'Elimination of all live lice and viable eggs from treated animals, as determined by inspection of sheep 52 weeks after treatment.' In current APVMA regulations the claim is applicable only to products applied off-shears or in short wool. Factors affecting the effective amount may include, for example, the parasite species to be treated and the development stages of the parasites, the wool length (see more details below) the type (e.g. species and breed, fine wool, coarse wool or hair wool), age, and condition of the infested animals; the environmental conditions (temperature, humidity), pharmacological considerations, such as the activity, efficacy, potency, selectivity, pharmacokinetic, and toxicologic profiles of the particular isoxazoline compound administered; and whether the isoxazoline compound being administered is part of a combination of active ingredients. Thus, the preferred amount of the compound according to this invention can vary.

A lice infestation is diagnosed by inspecting the fleece of sheep, especially the sheep suspected to be infested by lice with the naked eye. Lice can be found on most woolled areas of sheep. Densities of lice are highest along the sides and sometimes on the back of sheep. After shearing, a greater proportion of the population are found at sites on lower body regions such as under the neck, lower flanks and upper legs and in areas where the wool has not been closely shorn.

For determining the level of infestation of lice in sheep, the WAAVP guidelines 2006 (Holdsworth, PA et al: "World Association for the Advancement of Veterinary Parasitology guidelines for evaluating the efficacy of ectoparasiticides against biting lice, sucking lice and sheep keds on ruminants ", Veterinary Parasitology 136 (2006), 45-54) specify examination of 40 sites (partings), about 10 cm wide, in total per animal or 80 sites (partings), about 5 cm wide, per animal. The sites should be spaced so that they are representative of the full area of the body covered by the fleece on each side of the sheep. In addition, the APVMA recommends that you divide the examination sites equally on each side of the animal, that is, 20 (10 cm) or 40 (5 cm) sites should be examined on each side of the body (total = 40 (10 cm) or 80 (5 cm) sites). Furthermore, the APVMA recommends that the number of partings on each side of the body are distributed equally between the neck, shoulder, withers, rump and flank, that is, 4 (10 cm) or 8 (5 cm) partings at each location on each side of the body.

Preferably sheep are inspected for lice infestation and/ or existence of viable eggs 20 weeks after treatment.

The isoxazoline compounds for use in the current invention are known in the art and these compounds and their use as antiparasitics are described, for example, in US patent application US 2007/0066617, and International Patent applications WO 2005/085216, WO 2007/079162, WO 2009/002809, WO 2009/024541, WO 2009/003075, WO 2010/070068 and WO 2010/079077. This class of compounds is known to possess excellent activity against blood sucking ectoparasites, i.e. parasitic insect and acarids, such as ticks and fleas.

The composition, method or use according to the invention comprises an isoxazoline compound of the Formula (I), the isoxazoline compound is 4-[5-(3,5-dichlorophenyl)-5-trifluoromethyl-4,5-dihydroisoxazol-3-yl]-2-methyl-*N*-[(2,2,2-trifluoro-ethylcarbamoyl)-methyl]-benzamide (CAS RN 864731-61-3). This compound is also known as fluralaner (corresponding to 4-[5-(3,5-dichlorophenyl)-5-trifluoromethyl-4,5-dihydroisoxazol-3-yl]-2-methyl-N-[(2,2,2-trifluoro-ethylcarbamoyl)-methyl]-benzamide) and exists in various isomeric forms. A reference to an isoxazoline compound always includes all possible isomeric forms of such a compound. Unless otherwise stated, a compound structure that does not indicate a particular conformation is intended to encompass compositions of all the possible conformational isomers of the compound, as well as compositions comprising fewer than all the possible conformational isomers. In some embodiments, the compound is a chiral compound. In some embodiments, the compound is a non-chiral compound.

The method (or use) of this invention comprises to use racemic mixtures, for example, equal amounts of the enantiomers of such isoxazoline compounds as described above. In addition, the method of this invention includes isoxazoline compounds that are enriched compared to the racemic mixture in an enantiomer of Formula (I). Also included are the essentially pure enantiomers of such isoxazoline compounds.

When enantiomerically enriched, one enantiomer is present in greater amounts than the other, and the extent of enrichment can be defined by an expression of enantiomeric excess ("ee"), which is defined as (2x-I)-100 %, where x is the mole fraction of the dominant enantiomer in the mixture (e.g., an ee of 20 % corresponds to a 60:40 ratio of enantiomers). Preferably the compositions for use in the current invention have at least a 50 % enantiomeric excess; more preferably at least a 75 % enantiomeric excess; still more preferably at least a 90 % enantiomeric excess; and the most preferably at least a 94 % enantiomeric excess of the more active isomer. Of particular note are enantiomerically pure embodiments of the more active isomer.

Isoxazoline compounds as described above can comprise additional chiral centers. The method of this invention comprises racemic mixtures as well as enriched and essentially pure stereo configurations at these additional chiral centers.

For example, the isoxazolines comprise a chiral (or asymmetric) carbon at the 5-position of the isoxazoline ring. In some embodiments, for example, the chiral carbon has a left-handed (or "S" or "sinister") configuration. An example of such a compound is:

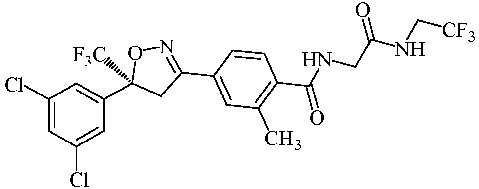

In other embodiments, the chiral carbon has a right-handed (or "R" or "rectus") configuration. An example of such a compound is:

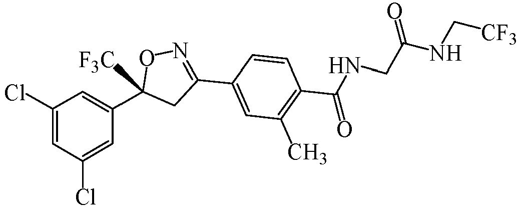

In one preferred embodiment the active enantiomer (s)-fluralaner is used.

Unless otherwise stated, an isoxazoline structure that does not indicate a particular conformation is intended to encompass compositions of all the possible conformational isomers of the isoxazoline, as well as compositions comprising fewer than all (e.g., just one of) the possible conformational isomers.

The reference to isoxazoline compound in this specification includes enantiomers, salts and solvates thereof that can be produced by conventional methods.

The term "salt" refers to salts prepared from pharmaceutically acceptable non-toxic bases or acids including inorganic or organic bases and inorganic or organic acids.

The term "solvate" is used herein to describe a molecular association comprising one or more pharmaceutically acceptable solvent molecules, e.g. water or ethanol. The term "hydrate" is used when said solvent is water.

Isoxazoline compounds of Formula (I) can be prepared according to one or other of the processes described e.g. in patent applications US 2007/0066617, WO 2007/079162, WO 2009/002809, WO 2009/080250, WO 2010/070068, WO 2010/079077, WO 2011/075591 and WO 2011/124998 or any other process coming within the competence of a person skilled in the art who is an expert in chemical synthesis. For the chemical preparation of the products of the invention, a person skilled in the art is regarded as having at her/his disposal, inter alia, the entire contents of "Chemical Abstracts" and of the documents cited therein.

In one embodiment with the current invention existing chewing lice infestation can be controlled, preferably eliminated within 15 days after administration.

In one embodiment with the current invention viable chewing lice eggs from treated animals cannot be found after evaluation of the sheep within 42 days after administration
In one embodiment with the current invention after a single administration no live lice and viable eggs can be found on treated animals 140 days after treatment.

It has been also discovered that the current invention can be also used to control (treat and/ or prevent infestation) resistant lice populations of sheep.

Resistance can be defined as exposure to a low dose of pesticide that enables some lice to survive, and these have genes that may allow them to survive higher doses that would normally kill all lice. Continued use of the same chemical or chemical group allows the resistant lice to survive, breed and increase in numbers until they make up the majority of the population. Sometimes, when resistance is present, treatment suppresses lice, but does not completely eradicate them. These suppressed infestations are difficult to detect and increase the chance of lice spreading between flocks.

The current invention can be used on sheep that are infested with lice resistant to any one of organophosphates, synthetic pyrethroids, neonicotinoids, spinosyn or benzoyl phenyl urea compounds and will still provide effective treatment/ protection.

In one embodiment the current invention is able to protect sheep for at least 2 weeks or 4 weeks from infestation by chewing lice
Lice infestations that can be treated/ prevented by the current invention can be present in various breeds of sheep including coarse wool sheep and fine wool sheep breeds such as Merino. It is known that sheep breeds have a different susceptibility to lice infestation. Merino breeds appear to be more susceptible than other breeds, but all breeds of sheep, including shedding breeds, such as Dorper and Damaras, can carry lice. The invention works in all such sheep breeds.

An important aspect of the current invention is that sheep with any length of wool can be successfully treated. This means an application within 24 hours after shearing- off shears, or during the time period >24 hours after shearing up to 6 weeks after shearing- short wool or > 6 weeks after shearing- long wool.

The current invention can be applied both on individual sheep that are lice infested or on sheep flocks with at least one sheep diagnosed with chewing lice infestation.

Up to now it has been easier to eradicate sheep lice on farms with one main shearing than it is on properties where mobs are shorn at different times (split shearing). The current invention allows successful control of lice infestations even under such conditions which is a major advantage for the management of sheep farms. Where split shearing occurs, in the past it was critical that treated mobs are kept separate from untreated sheep until the untreated sheep are treated at their next shearing, now all sheep can be treated at the same time irrespective of their shearing date.

In the past, flock management became more complicated where pregnant ewes or ewes with lambs at foot needed to be treated for lice. During this time, if there is contact with untreated lambs, there is the potential for lice to spread from the ewes to the lambs. Once the protective effect of the ewe treatment has worn off and if the lambs have not been treated, the lice can then spread back to the ewes to continue the infestation. This is because backline treatments and insect growth regulator products take some time to bring about the death of all lice. During this time, if there is contact with other untreated sheep, there is the potential for lice to spread.

Product labels of prior art products contain restrictions such as mandatory isolation periods (time when treated sheep must not be mixed with untreated sheep) that apply in this situation. Mandatory isolation periods vary between products but generally are 6 weeks.

Managing ewes with lambs at foot or ewes due to lamb soon after treatment is more complex. Especially in such situation it is very advantageous if a product protects sheep from re-infestation with chewing lice.

Typically effective (dosage) amount of isoxazoline compounds (especially fluralaner), are between 0.1 mg/kg bodyweight of the treated animal and 50 mg/kg bodyweight, or 0.25 mg/kg bodyweight to 10 mg/kg bw, or 0.5 mg/kg bw to 7.5 mg/kg bw, or 1 to 5 mg/kg bw. In one embodiment the effective dosage is 1.5 mg/kg bodyweight. In another embodiment the effective dosage is 0.5 mg/kg bodyweight, especially when sheep are treated off-shears. Especially preferred, in case of fluralaner, is a dosage between about 1.5 and about 3 mg/kg bodyweight of the animal that has been shown to eliminate lice infestation after single administration either <24 hours after shearing- (off-shears), >24 hours after shearing up to 6 weeks after shearing-(short wool )or > 6 weeks after shearing ( long wool).

Additionally it is possible that the single administration can be performed at different time points within the time frame indicated above without negative impact on the efficacy of the treatment, to allow flexibility e.g. when there is a need of treating large numbers of sheep. In one embodiment the single administration is within one week before shearing of the sheep.

It has been found that lice and eggs can be significantly reduced when the single oral administration of the isoxazoline compound, especially fluralaner was made 7 days before shearing, within 24 hours after shearing or 6 weeks after shearing.

In one embodiment a single dose of an effective amount of the isoxazoline compound is administered to a sheep, that has been diagnosed to be infested with chewing lice.

In one embodiment a single dose of an effective amount of the isoxazoline compound is administered to a sheep, that has been in contact with a sheep that has been diagnosed with a chewing lice infestation and is therefore at risk to be infested.

It has been surprisingly found that an extended duration of effective control, especially protection from new lice infestation is possible without the composition being an extended release composition to provide effective blood levels of the isoxazoline compound of formula (I) during the whole protection and/or treatment period. In one embodiment the protection period is 2 weeks. In another embodiment the protection period is 4 weeks.

The term "extended release" or "extended release composition" as used herein means a dosage form that is formulated in such a manner to make a pharmaceutically active ingredient to be available over an extended period of time due to the interaction of the formulation components/excipients in combination with the natural pharmacokinetic or pharmacodynamic characteristics of the active agent(s).

This definition is consistent with the use of the term known and accepted in the veterinary field as described in the article "Terminology Challenges: Defining Modified Release Dosage Forms in Veterinary Medicine" by Marilyn N. Martinez, Danielle Lindquist and Sanja Modric (Journal of Pharmaceutical Sciences, vol. 99, no. 8, August 2010).

A number of veterinary compositions are known to be suitable for oral administration to animals, but they vary for the different animal species. For sheep conventionally pharmaceutically active ingredients are administered orally as solids (e.g. tablets or boluses) or liquids, and via their feed or drinking water. In large sheep flocks the use of oral drenches is the most common oral dosage form, especially when administering anthelmintics.

Drenching means that a liquid, potentially slightly viscous composition comprising the active pharmaceutical ingredient and excipients is applied via the mouth with a specific drenching gun that dispenses an anthelmintic into the sheep's throat.

Oral drench compositions are in general solutions or suspensions and generally not more than 20 ml, preferably not more than 15 ml of such drench are applied per sheep.

In one embodiment a concentrated solution is used to administer the isoxazoline compound via drench application to sheep.

In another embodiment a suspension formulation is used for the drench application.

Excipients may comprise conventional non-active pharmaceutically and veterinarily acceptable components e.g. fillers, binders, flavoring agents, solvents, colorants, glidants, preservatives, especially solvents, surfactants, stabilizers, such as antioxidants. The pharmaceutical excipients are excipients with which the person skilled in the art is familiar, such as those which are described in the European Pharmacopoeia.

### Combinations

This invention is also directed to composition for use according to the invention comprising more than one pharmaceutically active ingredient, i.e. wherein the composition for oral administration comprises another active pharmaceutical ingredient.

Those of ordinary skill in the veterinary pharmaceutical arts will be entirely familiar with the identity of such active ingredients which may include, without limitation antiparasitics such as endoparasitics (including anthelmintics) and ecto-parasticides, hormones and/or derivatives thereof, and minerals and vitamins.

Especially preferred is a combination with selenium, especially as sodium selenate.

The active ingredients are preferably antiparasitics, more preferably endoparasiticides, preferably anthelmintics and are selected from the group consisting of avermectins (e.g., ivermectin, doramectin, abamectin); milbemycins (moxidectin and milbemycin oxime); pro-benzimidazoles (e.g., febantel, netobimin, and thiophanate); benzimidazole derivatives, such as a thiazole benzimidazole derivatives (e.g., thiabendazole and cambendazole), carbamate benzimidazole derivatives (e.g., fenbendazole, albendazole (oxide), mebendazole, oxfendazole, parbendazole, oxibendazole, flubendazole, and triclabendazole); imidazothiazoles (e.g., levamisole and tetramisole); tetrahydropyrimidine (morantel and pyrantel), salicylanilides (e.g., closantel, oxyclozanide, rafoxanide, and niclosamide); nitrophenolic compounds (e.g., nitroxynil and nitroscanate); benzenedisulfonamides (e.g., clorsulon); pyrazinoisoquinolines (e.g., praziquantel and epsiprantel); heterocyclic compounds (e.g., piperazine, diethylcarbamazine, and phenothiazine); dichlorophen, arsenicals (e.g., thiacetarsamide, melorsamine, and arsenamide); cyclooctadepsipeptides (e.g., emodepside); paraherquamides (e.g. derquantel); and amino-acetonitrile compounds (e.g. monepantel, AAD 1566); amidine compounds (e.g., amidantel and tribendimidin), including all pharmaceutically acceptable forms, such as salts, solvates or N-oxides.

Especially preferred are combinations with moxidectin, levamisole, fenbendazole, abamectin, monepantel, derquantel, oxfendazole, albendazole, closantel and combinations thereof e.g. as used in the following anthelminitic worm drench products that are commercially available in Australia: Trifecta, Zolvix Plus, Startect, Duocare LV, Cydectin SE, Q-Drench, Tridectin.

The composition according to the invention can be in one embodiment mixed with an existing commercial product before administration to an animal.

In an alternative embodiment the composition includes a (fixed) combination of an effective amount of an isoxazoline compound for controlling chewing lice and another active ingredient that controls a different parasite infestation, e.g. against parasitic helminths or alternatively fly strike. In a specific embodiment two isoxazoline compounds as described above are included that control different parasites.

Other combinations of the present invention can include ectoparasiticides such as insect or acarid growth regulators (AGRs or IGRs) such as e.g. lufenuron, diflubenzuron, novaluron, triflumuron, fluazuron, cyromazine, methoprene, pyriproxyfen dicyclanil etc., neonicotinoids, such as imidacloprid or thiacloprid or spinosyns, such as spinosad.

The amounts of each of the components in the final product may be varied considerably, depending upon the nature of the pharmaceutically active ingredients, the weight and condition of the subject treated, and the unit dosage desired. Those of ordinary skill in the art will be able to adjust dosage amounts for particular pharmaceutically active ingredients in the composition in light of the teachings of this disclosure.

The product according to the current invention conventionally further comprises physiologically acceptable formulation excipients known in the art e.g. as described in "Gennaro, Remington: The Science and Practice of Pharmacy" (20th Edition, 2000).

All such ingredients, carriers and excipients must be substantially pharmaceutically or veterinary pure and non-toxic in the amounts employed and must be compatible with the pharmaceutically active ingredients.

It is to be understood that a reference herein to a prior art document does not constitute an admission that the document forms part of the common general knowledge in the art.

In the paragraphs which follow and in the preceding description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

### Background example

### Pharmacokinetic Study (Topical, Oral and Subcutaneous Administration)

This study aimed to determine the pharmacokinetic profile of fluralaner following a single topical, oral or subcutaneous (SC) administration of fluralaner to sheep off-shears. The nominal dose was 3 mg/kg body weight (bw) for all formulations.

Four groups of 6 (3 females, 3 castrated males) young adult healthy Merino sheep received (within 24 h after shearing) a single administration of fluralaner at the nominal dose of 3 mg fluralaner per kg body weight either topically (Group 1: Pour-on 1; Group 2: Pour-on 2), orally (Group 3) or subcutaneously -SC (Group 4).

Blood samples were collected for 84 days after administration and plasma samples were analyzed for fluralaner.

Following topical administration, there was a rapid (over 5 days) and slow (over 12 days) increase to a peak concentration in Groups 2 and 1, respectively.

Then, the concentrations declined to values below 1 ng/mL at the late collection time points.

Following oral administration, rapid absorption of mean S-fluralaner was observed within the first day post-dosing. The peak was of ca. 100 ng/mL. Then, the concentration abruptly declined and by 21 days post-dosing, the concentration of all individuals was below the LLOQ.

Following SC administration, a slow increase of mean concentration to a peak concentration by 12 days post-dosing was observed. Then, the mean concentration declined progressively towards the last collection time point (i.e. 84 days post-dosing).

The pharmacokinetic parameters reflected the differences observed on the concentration-time profiles after topical, oral and SC administration. Higher AUC and Cₘₐₓ were observed in Group 3 (oral) together with shorter Tₘₐₓ while a higher MRTₗₐₛₜ was observed in Group 4 (SC).

The mean S-fluralaner concentration in blood is shown in Figure 2.

### Example 1

### Efficacy in sheep 9 months wool growth

In this study the efficacy of fluralaner when administered once orally at doses of 2.0 and 3.0 mg/kg body weight against sheep body lice *(Bovicola ovis)* infestation on Merino sheep with 9 months wool growth was evaluated. Plasma samples were also collected along with tissue samples at the end of the study.

Twenty-eight (28) medium wool Merino ewes with a heavy (>5 lice per 10 cm parting) sheep body lice infestation of diazinon resistant lice were divided into groups of seven animals such that each group had a similar mean lice burden.

Fluralaner was administered to sheep in Groups 2 and 3 on Day 0 at 2.0 and 3.0 mg fluralaner/kg bodyweight respectively. The dose was administered orally over the back of the tongue. Group 1 was left untreated as a negative control.

Pre-treatment lice counts were conducted on Day -1 and used to allocate sheep to treatment groups. Post-treatment lice counts were conducted 3, 7, 14, 21, 42, 70, 84, 112 and 140 days ± 1 day post-treatment.

Lice counts were performed according to the World Association for the Advancement of Veterinary Parasitology (W.A.A.V.P.) "Guidelines for evaluating the efficacy of ectoparasiticides against biting lice, sucking lice and sheep keds on ruminants" and the APVMA Pre-amble to these guidelines.

Twenty (20) sites on each side of the body were examined by opening the fleece about 10cm and counting all live lice seen. The total count from the forty (40) sites constituted the body count for each animal.

At the final lice count on Day 140 the number of sites examined was doubled to increase the sensitivity of the counting.

The sites examined were spaced so that they were representative of the full area of the body covered by the fleece on each side of the sheep. At each post-treatment lice count the number of viable lice eggs was also counted and recorded.

The percentage efficacy was calculated for each treatment group at each assessment time using the geometric mean lice counts and the Henderson-Tilton formula: $Percentage Efficacy = \left(1-\left(Ta/Ca\right)\times \left(Cb/Tb\right)\right) \times 100$ Where:
Ta = the mean number of lice on the treated group post treatment.
Ca = the mean number of lice on the negative control group post treatment.
Tb = the mean number of lice on the treated group before treatment.
Cb = the mean number of lice on the negative control group before treatment.

The treatment groups receiving 2 and 3 mg/kg dose rates reached 100% efficacy by 13 days post-treatment and maintained this level of efficacy until the last lice count on Day 140. There were no viable lice eggs detected at the 2 and 3 mg/kg dose rates from Day 41 until the last count on Day 140.

### Example 2

### Efficacy in sheep off-shears

In this study the efficacy of fluralaner when administered once orally at doses of 0.5, 1.0, 1.5 and 2.0 mg/kg body weight against sheep body lice *(Bovicola ovis)* infestation on Merino sheep off-shears was evaluated.

Thirty-five (35) Merino sheep with a heavy (>5 lice per 10 cm parting) sheep body lice infestation were allocated to five groups of seven animals such that each group had a similar mean lice burden. Fluralaner was administered to sheep in Groups 2, 3, 4 and 5 on Day 0 at 0.5, 1.0, 1.5 and 2.0 mg fluralaner/kg bodyweight respectively within 24 hours after shearing. The dose was administered orally over the back of the tongue. Group 1 was left untreated as a negative control.

Pre-treatment lice counts were conducted on Day -1 and used to allocate sheep to treatment groups. Post-treatment lice counts were/will be conducted 3, 7, 14, 21, 42, 84, 112 and 140 days ± 1 day post-treatment. Lice counts were performed as described in Example 1.

The 0.5, 1.0, 1.5 and 2.0 mg/kg dose rates reached 100% efficacy by 14 days post-treatment until the final count on Day 112. The 1.0, 1.5 and 2.0 mg/kg dose rates reached 100% efficacy by 7 days post-treatment and the efficacy of the 0.5 mg/kg dose rate was above 99%. There were no viable lice eggs detected at the 0.5, 1.0, 1.5 and 2.0 mg/kg dose rates from Day 21 until the final count on Day 112. There were no viable lice eggs detected at the 2.0 mg/kg dose rate from Day 14 until the final count on Day 112.

### Example 3

### Efficacy in sheep long wool

In this study the efficacy of fluralaner when administered once orally at doses of 1.5 and 2.0 mg/kg body weight against sheep body lice *(Bovicola ovis)* infestation on Merino sheep with 3, 6 and 9 months of wool growth was evaluated.

Thirty (30) Merino sheep per wool length with a moderate to heavy (1 - >5 lice per 10 cm parting) sheep body lice infestation were assigned to three groups of ten animals such that each group had a similar mean lice burden. Fluralaner was administered to sheep in each group at each wool length at 1.5 and 2.0 mg fluralaner/kg bodyweight respectively. The dose was administered orally over the back of the tongue. One group per wool length was left untreated as a negative control.

Pre-treatment lice counts were conducted on Day -1 and used to allocate sheep to treatment groups. Post-treatment lice counts were/will be conducted 7, 14, 21, 42, 90 and 140 days ± 1 day post-treatment or up until the next annual shearing, whichever occurs first. Lice counts were performed as described in Example 1.

The 1.5 and 2.0 mg/kg dose rates reached 100% efficacy by 14 days post-treatment at each wool length. There were no viable lice eggs detected at the 1.5 and 2.0 mg/kg dose rates from Day 42 at the 6- and 9-months wool lengths.

### Example 4

### Persistency Determination

In this study the persistent efficacy of fluralaner when administered once orally at doses of 1.5 and 2.0 mg/kg body weight against sheep body lice *(Bovicola ovis)* infestation on Merino sheep with 2 months wool growth was evaluated.

Forty-two (42) lice free Merino sheep were divided into six groups of seven animals such that each group had a similar mean body weight. Fluralaner was administered to sheep in two groups each at 1.5 and 2.0 mg fluralaner/kg bodyweight. The dose was administered orally over the back of the tongue. Two groups were left untreated as negative control groups.

From Day 0-7 and Day 7-14 post-treatment one group of untreated negative control sheep and one group of sheep treated at 1.5 and 2.0 mg/kg were challenged with lice by close confinement with lice infested sheep and manual transfer of lice. Following the challenges lice counts were conducted 7, 14, 21, 42 and 84 days ± 1 day post-challenge.

No lice or viable lice eggs were found on the sheep treated at the 1.5 and 2.0 mg/kg dose rates after either challenge 14, 21, 42 and 84 days ± 1 day post-challenge.

## Claims

1. Fluralaner or a salt or solvate thereof for use in a method of treatment or protection of sheep from *Bovicola ovis* chewing lice infestation after a single oral administration of a composition comprising an effective amount of fluralaner to sheep independent of its wool length.

2. The compound for use according to claim 1 wherein fluralaner is for the protection of sheep from *Bovicola ovis* chewing lice.

3. The compound for use according to claims 1 or 2 wherein the composition comprises another active pharmaceutical ingredient.

4. The compound for use according to any one of claims 1 to 3 wherein the effective amount of the fluralaner is between 0.5 and 3 mg/ kg bodyweight of the animal.

5. The compound for use according to any one of claims 1 to 4 wherein the composition is administered as oral drench.

6. The compound for use according to any one of claims 1 to 5 wherein the composition is administered once to a sheep mob with at least one sheep diagnosed with chewing lice infestation.

7. The compound for use according to claim 6 wherein the composition is administered once within 24 hours after shearing- (off shears).

8. The compound for use according to claim 6 wherein the composition is administered once during the time period >24 hours after shearing up to 6 weeks after shearing- (short wool).

9. The compound for use according to claim 6 wherein the composition is administered once > 6 weeks after shearing- (long wool).

10. The compound for use according to any one of claims 1 to 9 wherein the effective amount of fluralaner is sufficient to eliminate an existing chewing lice infestation within 15 days after administration.

11. The compound for use according to any one of claims 1 to 9 wherein the effective amount of fluralaner is sufficient to eliminate viable chewing lice eggs from treated animals within 42 days after administration.

12. The compound for use according to any one of claims 1 to 11 wherein the effective amount of fluralaner is sufficient after a single oral administration to eliminate all live lice and viable eggs from treated animals when investigated 140 days after treatment.

13. The compound for use according to any one of claims 1 to 12 wherein the effective amount of fluralaner is sufficient after a single oral administration to protect sheep for at least 2 weeks from chewing lice infestation.

14. The compound for use according to claim 13 wherein the effective amount of fluralaner is sufficient after a single oral administration to protect sheep for at least 6 weeks from chewing lice infestation.

15. The compound for use according to any one of claims 1 to 14 wherein the chewing lice are resistant to any one of organophosphates, synthetic pyrethroids, neonicotinoids, spinosyn or benzoyl phenyl urea compounds.

## Patentansprüche

1. Fluralaner oder ein Salz oder Solvat davon zur Verwendung in einem Verfahren zur Behandlung oder zum Schutz von Schafen vor einem Befall mit *Bovicola ovis*-Haarlingen nach einer einmaligen oralen Verabreichung einer Zusammensetzung, die eine wirksame Menge an Fluralaner umfasst, an Schafe unabhängig von ihrer Wolllänge.

2. Verbindung zur Verwendung nach Anspruch 1, wobei Fluralaner zum Schutz von Schafen vor *Bovicola ovis*-Haarlingen dient.

3. Verbindung zur Verwendung nach Anspruch 1 oder 2, wobei die Zusammensetzung einen weiteren pharmazeutischen Wirkstoff umfasst.

4. Verbindung zur Verwendung nach einem der Ansprüche 1 bis 3, wobei die wirksame Menge des Fluralaners zwischen 0,5 und 3 mg/kg Körpergewicht des Tieres liegt.

5. Verbindung zur Verwendung nach einem der Ansprüche 1 bis 4, wobei die Zusammensetzung als oral drench verabreicht wird.

6. Verbindung zur Verwendung nach einem der Ansprüche 1 bis 5, wobei die Zusammensetzung einmal an eine Schafherde mit mindestens einem Schaf, bei dem ein Haarlingsbefall diagnostiziert wurde, verabreicht wird.

7. Verbindung zur Verwendung nach Anspruch 6, wobei die Zusammensetzung einmal innerhalb von 24 Stunden nach der Schur - (frisch geschoren) verabreicht wird.

8. Verbindung zur Verwendung nach Anspruch 6, wobei die Zusammensetzung einmal während des Zeitraums von >24 Stunden nach der Schur bis zu 6 Wochen nach der Schur - (Kurzwolle) verabreicht wird.

9. Verbindung zur Verwendung nach Anspruch 6, wobei die Zusammensetzung einmal > 6 Wochen nach der Schur - (Langwolle) verabreicht wird.

10. Verbindung zur Verwendung nach einem der Ansprüche 1 bis 9, wobei die wirksame Menge an Fluralaner ausreicht, um einen bestehenden Haarlingsbefall innerhalb von 15 Tagen nach der Verabreichung zu beseitigen.

11. Verbindung zur Verwendung nach einem der Ansprüche 1 bis 9, wobei die wirksame Menge an Fluralaner ausreicht, um lebensfähige Haarlingseier von behandelten Tieren innerhalb von 42 Tagen nach der Verabreichung zu beseitigen.

12. Verbindung zur Verwendung nach einem der Ansprüche 1 bis 11, wobei die wirksame Menge an Fluralaner nach einer einmaligen oralen Verabreichung ausreicht, um alle lebenden Haarlinge und lebensfähigen Eier von behandelten Tieren zu beseitigen, wenn 140 Tage nach der Behandlung untersucht wird.

13. Verbindung zur Verwendung nach einem der Ansprüche 1 bis 12, wobei die wirksame Menge an Fluralaner nach einer einmaligen oralen Verabreichung ausreicht, um Schafe für mindestens 2 Wochen vor einem Haarlingsbefall zu schützen.

14. Verbindung zur Verwendung nach Anspruch 13, wobei die wirksame Menge an Fluralaner nach einer einmaligen oralen Verabreichung ausreicht, um Schafe für mindestens 6 Wochen vor einem Haarlingsbefall zu schützen.

15. Verbindung zur Verwendung nach einem der Ansprüche 1 bis 14, wobei die Haarlinge gegen einen beliebigen Vertreter von Organophosphaten, synthetischen Pyrethroiden, Neonicotinoiden, Spinosyn oder Benzoylphenylharnstoff-Verbindungen resistent sind.

## Revendications

1. Fluralaner ou un sel ou solvate de celui-ci pour utilisation dans une méthode de traitement ou de protection de moutons contre une infestation par des poux broyeurs Bovicola ovis après une administration orale unique d'une composition comprenant une quantité efficace de fluralaner à des moutons indépendamment de la longueur de leur laine.

2. Composé pour utilisation selon la revendication 1, dans lequel le fluralaner est destiné à la protection de moutons contre les poux broyeurs Bovicola ovis.

3. Composé pour utilisation selon la revendication 1 ou 2, dans lequel la composition comprend un autre ingrédient pharmaceutique actif.

4. Composé pour utilisation selon l'une quelconque des revendications 1 à 3, dans lequel la quantité efficace du fluralaner est comprise entre 0,5 et 3 mg/kg de poids corporel de l'animal.

5. Composé pour utilisation selon l'une quelconque des revendications 1 à 4, dans lequel la composition est administrée sous forme de drench oral.

6. Composé pour utilisation selon l'une quelconque des revendications 1 à 5, dans lequel la composition est administrée une fois à un troupeau de moutons ayant au moins un mouton diagnostiqué avec une infestation par des poux broyeurs.

7. Composé pour utilisation selon la revendication 6, dans lequel la composition est administrée une fois dans les 24 heures après la tonte - (immédiatement après la tonte).

8. Composé pour utilisation selon la revendication 6, dans lequel la composition est administrée une fois pendant la période de temps > 24 heures après la tonte jusqu'à 6 semaines après la tonte - (laine courte).

9. Composé pour utilisation selon la revendication 6, dans lequel la composition est administrée une fois > 6 semaines après la tonte - (laine longue).

10. Composé pour utilisation selon l'une quelconque des revendications 1 à 9, dans lequel la quantité efficace de fluralaner est suffisante pour éliminer une infestation existante par des poux broyeurs dans les 15 jours après l'administration.

11. Composé pour utilisation selon l'une quelconque des revendications 1 à 9, dans lequel la quantité efficace de fluralaner est suffisante pour éliminer les œufs viables de poux broyeurs chez des animaux traités dans les 42 jours après l'administration.

12. Composé pour utilisation selon l'une quelconque des revendications 1 à 11, dans lequel la quantité efficace de fluralaner est suffisante après une administration orale unique pour éliminer tous les poux vivants et les œufs viables chez des animaux traités lors d'un examen 140 jours après le traitement.

13. Composé pour utilisation selon l'une quelconque des revendications 1 à 12, dans lequel la quantité efficace de fluralaner est suffisante après une administration orale unique pour protéger des moutons pendant au moins 2 semaines contre une infestation par des poux broyeurs.

14. Composé pour utilisation selon la revendication 13, dans lequel la quantité efficace de fluralaner est suffisante après une administration orale unique pour protéger des moutons pendant au moins 6 semaines contre une infestation par des poux broyeurs.

15. Composé pour utilisation selon l'une quelconque des revendications 1 à 14, dans lequel les poux broyeurs sont résistants à l'un quelconque parmi les organophosphates, les pyréthrinoïdes de synthèse, les néonicotinoïdes, la spinosyne ou les composés de benzoylphénylurée.
